Europäisches Patentamt

European Patent Office

Office européen des brevets

(19)

(11) Publication number: **0 089 346**
**B1**

(12) **EUROPEAN PATENT SPECIFICATION**

(45) Date of publication of patent specification: **21.01.87**

(51) Int. Cl.⁴: **G 01 N 35/02, G 01 N 33/53**

(21) Application number: **82902706.9**

(22) Date of filing: **22.09.82**

(86) International application number:
**PCT/AU82/00158**

(87) International publication number:
**WO 83/01119 31.03.83 Gazette 83/08**

(54) **AUTOMATED IMMUNOASSAY SYSTEM.**

(30) Priority: **25.09.81 AU 913/81**

(43) Date of publication of application:
**28.09.83 Bulletin 83/39**

(45) Publication of the grant of the patent:
**21.01.87 Bulletin 87/04**

(84) Designated Contracting States:
**AT BE CH DE FR GB LI LU NL SE**

(56) References cited:
**AU-D-4 911 972**
**AU-D-5 881 780**
**AU-D-7 867 881**
**FR-A-2 389 134**
**FR-A-2 397 636**
**GB-A-2 014 727**
**US-A-3 785 533**
**US-A-3 859 051**

**issued 1979 (Amer Assoc. Clin-Chem, Winston-Salem,N.C.) D. Wagner, B. Alspector J. Feingers, A. Pick, "Direct Radioimmunoassay with Capillary Chromatography Tubes", see pages 1337 to 1339**

(73) Proprietor: **COMMONWEALTH SERUM LABORATORIES COMMISSION**
**45 Poplar Road**
**Parkville, Vic. 3052 (AU)**

(72) Inventor: **CHANDLER, Howard, Milne**
**Menzies Road**
**Kangaroo Ground, VIC 3097 (AU)**

(74) Representative: **Bizley, Richard Edward et al**
**BOULT, WADE & TENNANT 27 Furnival Street**
**London EC4A 1PQ (GB)**

(56) References cited:

**Biological Abstracts, Volume 69, No. 7, issued 1980 (Bio Sciences Information Service, Philadelphia, Penn USA) A.E. Buhl, L.M. Pasztor, J.A. Resko, "Sex steroids in guinea pig fetuses after sexual differentiation of the gonads"**

Courier Press, Leamington Spa, England.

## Description

This invention relates to an automated immunoassay system, in particular to a novel immunoassay system which is mechanically simple and compact, and hence relatively inexpensive, and yet is fully automated and capable of handling hundreds of unknown samples per day at a fraction of the cost per test when compared to existing automated systems. Although the system of this invention is suitable for radioimmunoassay (RIA) and other forms of immunoassay, it is particularly suitable for use in enzyme immunoassay by which the disadvantages of RIA including short shelf life of reagents, radioactivity, hazards, waste disposal problems and the like, can be avoided.

At present there are very few fully automated systems for heterogeneous enzyme immunoassays (EIAs), and those that do exist are both cumbersome and expensive. (See, for example, Oellerich M. "Enzyme Immunoassays in Clinical Chemistry: Present Status and Trends", J. Clin, Chem. Clin. Biochem. 1980; 18: 197—208). As a result, at the present time when heterogeneous enzyme immunoassays are used in hospitals and pathology laboratories, they are most commonly only semi-automated (requiring expensive equipment such as diluters, washers and plate readers), labour intensive and slow. There is therefore an urgent need for a compact, inexpensive, fully automated heterogeneous enzyme immunoassay system capable of handling hundres of samples per day.

French Patent Application No. 2389134 (Seroa) relates to an analytical method for chemicals, biochemicals, immunochemicals and their analogues which uses capillary tubes. The advantage of using very small reagent quantities is discussed. However, each analytical stage has to be carried out by hand, a delicate and time-consuming task. The small quantities involved in capillary reactions are also subject to human inaccuracies. French Patent Application No. 2397636 (Gist-Brocades N.V.) also discusses the use of capillary tubes in chemical analysis. Again, however, there are a large number of manual stages involved. There is no disclosure of an automated analytical system using such capillary tubes, and it seems apparent from the detailed techniques involved that an automated system would be beneficial.

It has now been discovered that a simple and highly effective system can be based on the use of capillary tubes as the solid phase in a heterogeneous enzyme immunoassay system.

According to a first aspect of this invention, there is provided apparatus for performing automated heterogeneous immunoassays for the detection or determination of antigenic or heptenic substances or antibodies in a plurality of samples which comprises:—

(a) a plurality of capillary tubes, each of said capillary tubes having antibodies or antigenic or haptenic substances attached to the internal surface thereof;

(b) means for passing each of said capillary tubes, in sequence, to a plurality of operation stations;

(c) means at a first operation station for admitting individual samples, in sequence, to each of said capillary tubes as it is passed to said first operation station;

(d) means at one or more subsequent operation station(s) for admitting immunoassay reagent to each of said capillary tubes as it is passed to said subsequent operation station(s); and

(e) means at a final operation station for detecting or determining the result of the immunoassay in each of said capillary tubes as it is passed to said final operation station.

In one embodiment of this first aspect of the present invention, there is provided apparatus for performing automated heterogeneous enzyme immunoassays for the detection or determination of antigenic or haptenic substances or antibodies in a plurality of samples which comprises:—

(a) a plurality of capillary tubes, each of said capillary tubes having antibodies or antigenic or haptenic substances attached to the internal surface thereof;

(b) means for passing each of said capillary tubes, in sequence, to a plurality of operation stations;

(c) means at a first operation station for admitting individual samples, in sequence, to each of said capillary tubes as it is passed to said first operation station;

(d) means for admitting enzyme conjugate to each of said samples, said enzyme conjugate being added either to each of said individual samples before said sample is admitted to one of said capillary tubes, or to each of said individual samples in one of said capillary tubes at a second operation station;

(e) means at a third operation station for admitting enzyme substrate to each of said capillary tubes as it is passed to said third operation station; and

(f) means at a fourth operation station for detecting or determining enzymic action, if any, on said enzyme substrate in each of said capillary tubes.

According to a second aspect of this invention, there is provided a method for performing automated heterogeneous immunoassays for the detection or determination of antigenic or haptenic substances or antibodies in a plurality of samples which comprises:—

(a) providing a plurality of capillary tubes, each of said capillary tubes having antibodies or antigenic or haptenic substances attached to the internal surface thereof;

(b) passing each of said capillary tubes, in sequence, to a plurality of operation stations;

(c) admitting individual samples, in sequence, to each of said capillary tubes at a first operation station;

(d) admitting immunoassay reagent to each of said capillary tubes at one or more subsequent operation stations; and

(e) detecting or determining the result of the immunoassay in each of said capillary tubes at a final operation station.

In one embodiment of this second aspect of the invention, there is provided a method for performing automated heterogeneous enzyme immunoassays for the detection or determination of antigenic or haptenic substances or antibodies in a plurality of samples which comprises:—

(a) providing a plurality of capillary tubes, each of said capillary tubes having antibodies or antigenic or haptenic substances attached to the internal surface thereof;

(b) passing each of said capillary tubes, in sequence, to a plurality of operation stations;

(c) admitting individual samples, in sequence, to each of said capillary tubes at to a first operation station;

(d) admitting enzyme conjugate to each of said samples, either before said sample is admitted to a capillary tube or at a second operation station;

(e) admitting enzyme substrate to each of said capillary tubes at a third operation station; and

(f) detecting or determining enzymic action, if any, on said enzyme substrate in each of said capillary tubes at a fourth operation station.

It will be apparent from the above broad description, that the present invention encompasses assay procedures in which the enzyme conjugate is mixed with the sample before it is added to the capillary tube (for example in the so-called "competitive" or "inhibition" assays—see A(c) below) as well as procedures in which the sample is first added to the capillary tube and enzyme conjugate then added (for example, in the so-called "sandwich" assays—see A(a) and B(a) below). If desired, additional operation stations may also be included to enable the admission of further reagent(s) at appropriate points in the assay procedure. Such further reagents may include, for example, a second antibody where a double antibody type of assay procedure is adopted (see A(b) and B(b) below). In this way, the apparatus and method of the present invention may be adapted to perform a wide variety of assay procedures. The following are illustrative, but by no means limiting, of the types of procedures which may be performed:

A: Antigen detection, e.g. hepatitis, digoxin
(a) Sandwich antigen assay:
1. Solid phase: Tube—Anti-hepatitis Ab
2. Specimen: ± Heptatitis subunit or virus
3. Conjugate: Anti-hepatitis Ab—enzyme

(b) Double antibody sandwich antigen assay:
1. Solid phase: Tube—Anti-hepatitis Ab Type 1 (e.g. sheep antibody)
2. Specimen: ±Hepatitis subunit or virus
3. Second Antibody: Anti-hepatitis Ab Type 2 (e.g. rabbit antibody)
4. Conjugate: Anti-type 2 Ab—enzyme

(c) Competitive antigen assay:
1. Specimen: ±Digoxin
2. Conjugate: Anti-digoxin Ab—enzyme
3. Solid phase: Tube—Digoxin.

(Note: In this assay specimen and conjugate are mixed and incubated prior to addition to tube).

B: Antibody detection, e.g. tetanus, rubella
(a) Sandwich antibody assay:
1. Solid phase: Tube—Tetanus Ag
2. Specimen: ±Anti-tetanus Ab (Human)
3. Conjugate: Anti-human Ab—enzyme.

(b) Double antibody sandwich antibody assay:
1. Solid phase: Tube—Tetanus Ag
2. Specimen: ±Tetanus Ab (Human)
3. Second Antibody: Anti-human Ab Type 2 (e.g. sheep antibody against human antibody)
4. Conjugate: Anti-type 2 Ab—enzyme.

Further operation stations may also be interspersed between the first and second operation stations and the second and third operation stations, and at each of these further operation stations may be located means for washing and vacuum drying each of said capillary tubes before it is passed to the above mentioned second operation station, or third operation station, respectively. Preferably, the various operation stations are suitably located so that appropriate incubation periods are provided as each capillary tube passes from one operation station to the next.

Preferably, the capillary tubes are mounted on a carrier in the form of a spool or carousel having the capillary tubes mounted vertically around the circumference thereof, each capillary tube then forming the reaction vessel (and solid phase) for a single EIA test. In one presently preferred embodiment of such a carrier, the carousel is provided with an appropriate number of outwardly facing slots or detents disposed around its periphery, each slot or detent being dimensioned to receive a capillary tube as a "push-fit" therein, and to releasably retain the tube until it is forced outwardly of the slot. In this way, the capillary tubes may be loaded into the periphery of the carousel, held therein during the immunoassay and later removed to enable fresh tubes to be loaded into the carousel. Suitable loading and unloading means may be provided as described hereinafter. By way of example, a carousel having diameter of approximately 6 cm might be provided with a capacity to hold 60 capillary tubes around the circumference thereof, whilst a carousel of diameter of approximately 40 cm may have a capacity of 240 tubes. Alternatively, however, the carrier may be in some other configuration, for example, in the form of an elongate, flexible belt somewhat similar to an "ammunition belt" configuration, again having the capillary tubes mounted vertically therein.

In the embodiment of this apparatus in which the carrier is in the form of a carousel, the automated EIA apparatus conveniently includes means for rotating this carousel in a stepwise manner so that each capillary tube at the circumference of the carousel passes, in sequence, the various operation stations which are located around the perimeter of the spool.

The capillary tubes which are used in the apparatus of the present invention may be made of any suitable material such as glass, polyvinyl chloride, polystyrene or other suitable plastics materials. By way of example, the tubes may be 1.5 cm—2.0 cm in length, and have an internal diameter of about 1 mm and an external diameter of about 2 mm. Such tubes have a capacity of around 10—15 µl. More generally, however, capillary tubes having a capacity in the range of from 1 to 30 µl may be used in the apparatus of the invention.

Further details of the apparatus of this invention will be apparent from the following description of a preferred embodiment of the invention which is illustrated, by way of example, in the accompanying drawings. In the drawings:—

Figure 1 is a diagrammatic representation of a preferred embodiment of the apparatus of the invention indicating the cycle of operations of this embodiment;

Figures 1a and 1b are top and side elevations, respectively (not to scale), of a schematic representation of a carousel device which is preferred for use in the automated apparatus;

Figure 2 is a perspective view of one embodiment of the apparatus of this invention; and

Figure 3 is an enlarged elevational view in section, of a head device for addition of reagent or wash fluid to the capillary tubes together with a vacuum device for drying the tubes.

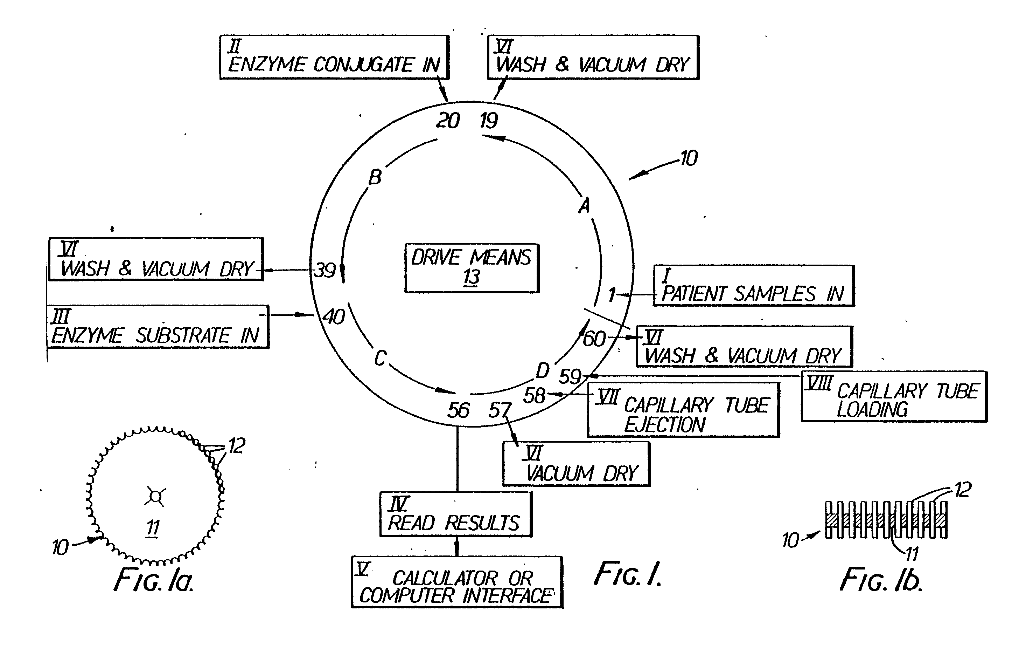

Referring firstly to Figures 1a and 1b the device 10 comprises a carrier 11 in the form of a carousel having the capacity to hold a plurality of capillary tubes 12 (not all of which are shown) vertically clipped into slots or detents around the circumference of the carousel. As previously mentioned, about 240 such capillary tubes might be held around a spool of diameter of about 40 cm. The internal surface of each of these capillary tubes is coated with antibody or antigenic or haptenic substance by techniques known per se, including adsorption or covalent bonding.

Turning now to Figure 1, there is schematically depicted apparatus in accordance with the preferred embodiment of the present invention which comprises a device 10 of the type shown in Figures 1a and 1b, and drive means 13 for stepwise rotation of the device 10 so that each of the capillary tubes on the circumference of the device 10 passes, in sequence, the operation stations positioned around the circular path of each capillary tube. Drive means 13 may, for example, comprise a geared motor controlled by a microprocessor to achieve this stepwise rotation. Accurate location of the capillary tubes at each of the operation stations can be effected by means of a detent mechanism operating on unoccupied or unfilled slots or detents in the periphery of the carousel. Further details of each of these operation stations will become apparent from the description of the operation of the apparatus below.

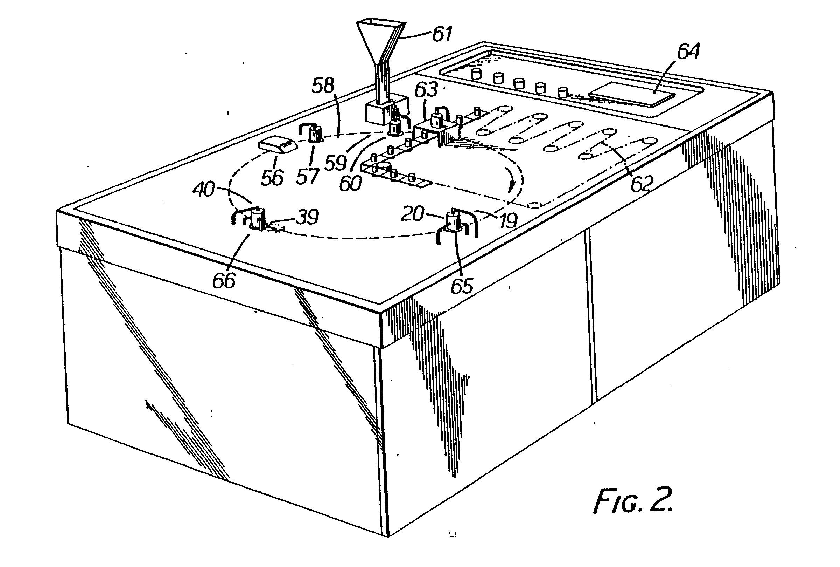

As shown in Figure 2, means including hopper 61 are provided to supply capillary tubes to tube loading station 59 (see Figure 1), the tubes falling from the hopper under gravity and being forced along a guide path and into unoccupied slots or detents on the periphery of the carousel in the vertical plane by a rotating resilient wheel, the vertical position of the tubes in the carousel being controlled by suitably positioned guide plates.

For any given test, it will be readily recognised that the capillary tubes 12 of this device 10 will be coated on the internal surfaces thereof with antibody or antigenic or haptenic substances appropriate for the particular test. Loading of the unknown samples at the first operation station I at position 1 commences the test procedure. As the apparatus of this invention may be used with any immunoassay procedure, the samples may, for example, be serum, plasma, urine or saliva, or toxins or drugs in solution. Whilst the loading of the tubes may be by any suitable means, it has been found that the use of small sample dispensing cups mounted in a separate cup conveyor belt 62 as shown in Figure 2 enables a plurality of samples to be quickly and readily loaded into the capillary tubes 12. Preferably, each unknown sample to be tested is contained in a sample dispensing cup on the separate conveyor belt 62, each of the sample dispensing cups having a small hole formed in the base thereof. The capillary tube to be loaded is brought into contact with this hole in the base of the cup, for example, by hydraulically lowering the cup under the operation of a cam or microprocessor, at loading bridge 63. The sample which has previously been retained in the cup notwithstanding the small hole in the base thereof, passes by capillary action into the tube. In this way, only a very small volume of each sample is required for testing, and the need for complex sampling and sample loading equipment is avoided. After use, each sample dispensing cup in belt 62 may be removed and replaced by a further cup containing a further unknown sample.

After a sample has been loaded into a first capillary tube, drive means 13 advances the device 10 by one step so that a new capillary tube is brought to the first operation station I and at the same time another sample in a further sample dispensing cup is brought into position at loading bridge 63 for loading into this new capillary tube in the same way as previously described. The loading of subsequent capillary tubes proceeds similarly. It will, of course, be apparent that as the loading of the capillary tubes with sample proceeds, the first capillary tube is being advanced stepwise towards the second operation station II.

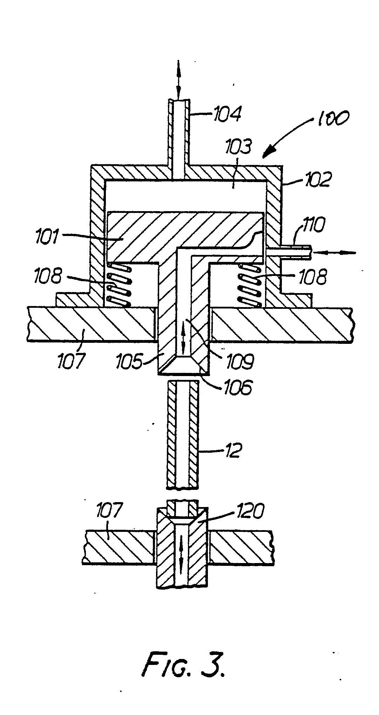

In the cycle of operations depicted in Figure 1, before reaching the second operating station II, the first capillary tube reaches an intermediate operation station VI at position 19 at which the tube is positioned over a vacuum outlet. The contents of the tube are evacuated and wash buffer metered through the tube from above. Figure 3 shows in detail a suitable head device 100 for addition of wash fluid to the capillary tubes, as well as a suitable vacuum device 120 for drying the tubes. As shown in this Figure, the device 100 comprises a hydraulically operated piston device 101 mounted in housing 102 and arranged to be moved toward the upper end of capillary tube 12 by hydraulic fluid admitted under pressure to chamber 103 formed by the piston 101 and housing 102 via line 104. Piston 101 is provided with an extension 105 having a concave recess 106 at the extremity thereof within which one end of tube 12 is received as shown. Extension 105 of the piston extends through an appropriate aperture in the support plate 107 and piston 101 is returned to its starting position under action of return spring 108 on release of the hydraulic pressure in chamber 103. Piston 101 and extension 105 are provided with a fluid passageway 109, extending from recess 106 to inlet line 110, through which appropriate wash fluid may be supplied from a metering pump, (not shown) to capillary tube 12 when the tube is engaged in recess 106. Suitable timing means such as a microprocessor or a mechanical cam device are, of course, provided to supply hydraulic fluid under pressure to chamber 103 and thus move piston 101 into engagement with tube 12, and to then supply wash fluid via passageway 109, before the piston returns to its starting position preparatory to repeating the cycle when another capillary tube 12 is moved into position.

Figure 3 also shows a vacuum device 120 positioned at the lower end of tube 12. This device is similar to head device 100 described above with the exception that the passageway extending to the recess is connected to a vacuum. Timing of the operation of device 120 may be arranged to be prior to, simultaneous with, or slightly behind in time, the operation of device 100. In this manner, tube 12 is emptied and vacuum dried before, simultaneously with or slightly after wash fluid is supplied.

Returning now to Figure 1, after washing and drying, the first capillary tube continues to advance stepwise and is then passed to the second operation station II at position 20 where it receives a metered quantity of enzyme conjugate, for example from a micropump, an automatic pipette or similar metering device. Preferably this reagent is supplied by means of a head device similar in construction and operation to device 100 previously described. In appropriate situations where a wash station is immediately adjacent a reagent addition station, for example at positions 19, 20 and positions 39, 40 in Figure 1, a single device may be used to simultaneously add wash solution to one capillary tube and to add reagent to the immediately adjacent capillary tube (see devices 65 and 66 in Figure 2).

The first capillary tube then advances to a further intermediate operation station VI at position 39 where the capillary tube is again vacuum dried and washed in the same manner as previously described. The tube then advances to the third operation station III at position 40 at which a metered quantity of enzyme substrate is added to the capillary tube. Once again, this may, for example, be effected by means of a micropump, an automatic pipette or similar metering device operating through a head device as previously described.

The tube again advances to the fourth operation station IV at position 56 at which the result of the test which has been·performed in that tube is read, for example by a colorimetric reading taken either through or along the length of the capillary tube. Such a reading may, for example, be accomplished by the use of chopped light from a suitable lamp focused through the capillary tube to impinge on a photodetector. The readout from the colorimeter is passed to a calculator or computer interface V, before being displayed at display panel 64 (Figure 2) or recorded by suitable print-out means.

Suitable switching or other actuating means will, of course, be provided to ensure that the metering or other device at each operating station is actuated to operate on each capillary tube as it arrives at that station.

From the above description, it will be appreciated that each capillary tube of the device 10 passes through three incubation phases, the first incubation phase being indicated in Figure 1 by the letter A and extending from positions 2 to 18; the second incubation period being indicated by the letter B and extending from positions 21 to 38; and the third incubation period being indicated by the letter C and extending from positions 41 to 55. In addition, as each of the capillary tubes advances stepwise, it passes through the various operation stations and the incubation periods in sequence.

If desired, when commencing the cycle of operations with a given device 10, the operation of each of the operation stations may be arranged to commence as the first capillary tube is advanced from station to station by means of a microswitch positioned at each of the operation stations. The advancing capillaries would activate each operation, for example by activating the circuit of a solenoid-operated micropump for the particular reagent addition required. The passage of the final capillary tube of the device 10 would allow the inactivation of each operation after that final capillary tube has passed the particular operation station.

On completion of the reading of the test in each tube, the tube passes to station VI where it is vacuum dried before passing to tube ejection station VII where it is forced outwardly from the slot or detent in which it was held, for example by means of an unloading device angled to the periphery of the carousel and positioned to engage each capillary tube in turn and force it outwardly.

In a modified embodiment, not shown, the device 10 could be replaced by a toothed wheel of similar size around which would be fed capillary tubes in an "ammunition belt" configuration, the capillary tubes and belt being discarded after use. It will, of course, be appreociated that the same advantages arise from the use of the present invention in this modified embodiment.

The cycle of operation which is described above with reference to Figure 1 of the drawings illustrates the sequence of steps which are used with assays of the type used for quantitation of rubella and tetanus immunity. For each of these assays, periods of approximately ten minutes are used for the incubation periods, A, B and C, so that, after completing the first cycle of 30 minutes, tests may be completed at the rate of about 120 per hour using a device 10 having 60 capillary tubes mounted thereon. If the diameter of the spool is increased to around 30 cm, the throughput can be increased to around 600 tests per hour. Of course, by suitably arranging the positions of the various operation stations and the speed at which the capillary tubes are passed to each operation station, the various incubation periods which are required for other types of assays could be accommodated. Overall, however, the use of capillary tubes in accordance with the present invention has a very significant advantage in that it enables very short incubation periods to be utilised because of the large reactive surface area and small reaction volume of the tube. The short incubation periods therefore make possible a large throughput of tests utilising the apparatus of the present invention.

A further and most significant advantage of the use of capillary tubes in accordance with the present invention arises from the fact that the capillaries require only very small quantities of the reagents, (for example in the range of 1 to 30 µl, particularly of the order of 10 to 15 µl) and so the amounts of reagents which are required to conduct a series of tests in this apparatus are minimised. The capillary tubes, of course, form open-ended reaction vessels and will automatically pick up and hold their own volume of reagent which may later be emptied simply by passing the tube over a vacuum outlet. As a result, the equipment for loading and washing the tubes may be simple, compact and inexpensive. In contrast, in existing automated EIA systems, reagents in the 100 to 200 µl quantities are metered and dispensed into tubes which, after incubations ranging from 30 minutes upward in a heated water bath, must be evacuated by retractable needles inserted into the tubes. Equipment for these tests is therefore complex, cumbersome and expensive.

It will be appreciated that variations to the sequence of operating stations illustrated in Figure 1 may be made as desired in order to accommodate alternative assay procedures.

Thus, in a "competitive" or "inhibition" type of assay, the operation station at which the enzyme conjugate is added to the individual samples is relocated so as to operate at a point on the cup conveyor belt or turntable prior in sequence to addition of the samples to the capillary tubes. In this modification, the conjugate can be added to the samples and incubated prior to addition of the individual mixtures to the tubes. Similarly, additional operation stations may be included at appropriate positions in the cycle of operation for addition of a second antibody in the "double antibody" type of assay.

In one particularly preferred embodiment of the apparatus of this invention, the various operation stations at which the steps of the method are performed are so constructed that the locations of the individual operation stations may be adjusted as desired to different points either along the sample path (for example, where enzyme conjugate is to be mixed and incubated with the samples prior to addition to the capillary tubes) or along the path of the capillary tubes themselves (for example, where the samples are added to the capillary tubes prior to addition of enzyme conjugate thereto). Such adjustment enables appropriate incubation times to be readily and easily selected for the various steps of the test sequence. The adjustment of location of the various operation stations may, for example, be achieved by providing a continuous rod or other support alongside the sample path and/or the path of the capillary tubes, and by so constructing the various operation stations that they can be located at any desired position along this support.

The following examples illustrate the use of the apparatus and method of the present invention in heterogeneous enzyme immunoassays:

Example 1
Estimation of tetanus immunity in blood from human patients

This estimation is performed in apparatus of the type illustrated in the accompanying drawings. The capillary tubes are of 1.5 cm length and internal diameter of 1.00 mm, having a capacity of about 10—15 µl. The following test system is used:

Solid phase: Tube—Tetanus Ag
Specimen: ±Anti-tetanus Ab (IgG)
Conjugate: Anti-human IgG—urease
Substrate/Indicator: Urea solution containing Bromocresol purple.

The specimens (human blood) were diluted 1:2 in anticoagulant buffer prior to testing. The cycle of operations is as shown in Figure 1, and the tests performed at room temperature with a 10 minute incubation period between addition of specimens to the tubes and washing the vacuum drying, and again between addition of conjugate and washing and drying, and between addition of substrate/indicator and reading of results.

The results obtained give good correlation when ranked against known standards.

Example 2

Estimation of digoxin in human serum

This estimation is performed using the same capillary tubes as described above, but in a "competitive" type assay in which the cycle of operations illustrated in Figure 1 is modified so that the enzyme conjugate is added to the specimen and incubated prior to addition of the resulting mixtures to the tubes. Again, 10 minute incubation periods at room temperature are used throughout the tests. The following test system is used:

Specimen: ±Digoxin

Conjugate: Anti-digoxin Ab—urease

Solid Phase: Tube—Digoxin

Substrate/Indicator: Urea solution containing Bromocresol purple.

This system has been found to give a very clear detection of digoxin in the 0.64—6.4 n moles/litre range in human serum.

Other enzymes may of course be used in place of urease as described above, and a number of marker enzymes for use in enzyme immunoassays have previously been described (see Oellerich: Enzyme Immunoassays in Clinical Chemistry, supra.). The use of enzymes such as urease, horse radish peroxidase, alkaline phosphatase and β-galactosidase is, however, preferred as these enzymes can be determined using indicators which provide results which can be read spectrophotometrically. It will be understood, however, that other enzymes and other detection methods, e.g. fluorimetry, luminescence measurement, turbidimetry, potentiometry and thermometry, may be adopted if desired.

Those skilled in the art will appreciate that the invention described herein is susceptible to other variations and modifications other than those specifically described without departing from the scope of the claims.

**Claims**

1. Apparatus for performing automated heterogeneous immunoassays for the detection or determination of antigenic or haptenic substances or antibodies in a plurality of samples which comprises:—

(a) a plurality of capillary tubes (12) each of said capillary tubes having antibodies or antigenic or haptenic substances attached to the internal surface thereof;

(b) means (10, 13) for passing each of said capillary tubes, in sequence to a plurality of operation stations;

(c) means at a first operation station (I) for admitting individual samples, in sequence, to each of said capillary tubes as it is passed to said first operation station;

(d) means (65, 66) at one or more subsequent operation station(s) (II, III) for admitting immunoassay reagent to each of said capillary tubes as it is passed to said subsequent operation station(s); and

(e) means at a final operation station (IV, V) for detecting or determining the result of the immunoassay in each of said capillary tubes as it is passed to said final operation station.

2. Apparatus as claimed in claim 1, wherein said capillary tubes (12) are mounted on a carrier (11), said carrier comprising a spool or carousel having said capillary tubes mounted vertically around the periphery thereof.

3. Apparatus as claimed in claim 2, wherein said means for passing (10, 13) each of said capillary tubes to a plurality of operations comprises means (13) for rotating said spool or carousel in a stepwise manner.

4. Apparatus as claimed in claim 2 or claim 3, wherein each of said capillary tubes is releasably retained by frictional engagement in an outwardly directed slot or detent at the periphery of said spool or carousel.

5. Apparatus as claimed in claim 4, further comprising means (61) for loading individual capillary tubes into individual slots or detents of said spool or carousel prior to passage of said tubes to said operation stations, and means for unloading said capillary tubes from said spool or carousel on completion of the immunoassay.

6. Apparatus as claimed in any one of claims 1 to 5 further comprising means to supply said plurality of samples, said means comprising a plurality of sample dispensing cups mounted in cup conveyor means (62) and drive means to advance each of said sample dispensing cups in turn to said first operation station (I), each of said sample dispensing cups having a small aperture in the base thereof and the sample being retained therein by capillary action, the apparatus further comprising loading means (63) at said first operation station to contact each capillary tube in turn with the base of a respective one of said dispensing cups so that the sample in said dispensing cup is loaded into said capillary tube.

7. Apparatus as claimed in any one of claims 1 to 6, further comprising washing (100) and/or vacuum drying (120) means positioned at selected points (VI) along the pathway of the capillary tubes intermediate said operation stations.

8. Apparatus as claimed in claim 7, wherein each of said washing and/or vacuum drying means includes a piston device (101) movable within a housing (102) to contact each of said capillary tubes in turn as it is passed to said washing and/or vacuum drying means to admit washing fluid to said tube and/or to remove washing and test fluids from said tube.

9. Apparatus as claimed in any one of claims 1 to 8, wherein each of said capillary tubes has a capacity of 1—30 µl, preferably 10—15 µl.

10. Apparatus for performing automated heterogeneous enzyme immunoassays for the detection or determination of antigenic or haptenic substances or antibodies in a plurality of samples which comprises:—

(a) a plurality of capillary tubes (12), each of said capillary tubes having antibodies or antigenic or haptenic substances attached to the internal surface thereof;

(b) means (13) for passing each of said capillary tubes, in sequence, to a plurality of operation stations;

(c) means (63) at a first operation station (I) for admitting individual samples, in sequence, to each of said capillary tubes as it is passed to said first operation station;

(d) means (65) for admitting enzyme conjugate to each of said samples, said enzyme conjugate being added either to each of said individual samples before said sample is admitted to one of said capillary tubes, or to each of said individual samples in one of said capillary tubes at a second operation station (II);

(e) means (66) at a third operation station (III) for admitting enzyme substrate to each of said capillary tubes as it is passed to said third operation station; and

(f) means at a fourth operation station (IV) for detecting or determining enzymic action, if any, on said enzyme substrate in each of said capillary tubes.

11. A method for performing automated heterogeneous immunoassays for the detection or determination of antigenic or haptenic substances or antibodies in a plurality of samples which comprises:—

(a) providing a plurality of capillary tubes, each of said capillary tubes having antibodies or antigenic or haptenic substances attached to the internal surface thereof;

(b) passing each of said capillary tubes, in sequence, to a plurality of operation stations;

(c) admitting individual samples, in sequence, to each of said capillary tubes at a first operation station;

(d) admitting immunoassay reagent to each of said capillary tubes at one or more subsequent operation stations; and

(e) detecting or determining the result of the immunoassay in each of said capillary tubes at a final operation station.

12. A method for performing automated heterogeneous enzyme immunoassays for the detection or determination of antigenic or haptenic substances or antibodies in a plurality of samples which comprises:—

(a) providing a plurality of capillary tubes, each of said capillary tubes having antibodies or antigenic or haptenic substances attached to the internal surface thereof;

(b) passing each of said capillary tubes, in sequence, to a plurality of operation stations;

(c) admitting individual samples, in sequence, to each of said capillary tubes at a first operation station;

(d) admitting enzyme conjugate to each of said samples, either before said sample is admitted to a capillary tube or at a second operation station;

(e) admitting enzyme substrate to each of said capillary tubes at a third operation station; and

(f) detecting or determining enzymic action, if any, on said enzyme substrate in each of said capillary tubes at a fourth operation station.

**Patentansprüche**

1. Vorrichtung zur Durchführung von automatisierten, heterogen Immunoassays für den Nachweis oder die Bestimmung von antigenischen oder haptenischen Substanzen oder Antikörpern in einer Mehrzahl von Proben, umfassend:

(a) eine Mehrzahl von Kapillarröhrchen (12), wobei jedes der Kapillarröhrchen Antikörper oder antigenische oder haptenische Substanzen an der Innenoberfläche angebracht enthält,

(b) Einrichtungen (10, 13), um jedes der Kapillarröhrchen in einer Reihenfolge zu einer Mehrzahl von Betriebsstationen zu leiten,

(c) Einrichtungen an einer ersten Betriebsstation (I), um die einzelnen Proben in Reihenfolge jedem der Kapillarröhrchen, wenn diese die erste Betriebsstation passieren, zuzuführen,

(d) Einrichtungen (65, 66) in einer oder mehreren nachfolgenden Betriebsstationen (II, III), um ein Immunoassay-Reagens zu jedem der Kapillarröhrchen, wenn es die nachfolgenden Betriebsstationen passiert, zuzuführen, und

(e) Einrichtungen für eine letzte Betriebsstation (IV, V) zum Nachweis oder Bestimmen der Ergebnisse des Immunoassays in jedem der Kapillarröhrchen, wenn diese die letzte Betriebsstation passieren.

2. Vorrichtung gemäss Anspruch 1, in welcher die Kapillarrohre (12) auf einem Träger (11) befestigt sind, wobei der Träger aus einer Spule oder einem Karussel, in welchem die Kapillarröhrchen vertikal um dessen Peripherie angeordnet sind, umfasst.

3. Vorrichtung gemäss Anspruch 2, worin die Einrichtungen zum Passieren (10, 13) jedes der Kapillarröhrchen zu einer Mehrzahl von Betriebsstationen Einrichtungen (13) zum Rotieren der Spule oder des Karussels in einer stufenweisen Form umfasst.

4. Vorrichtung gemäss Anspruch 2 oder Anspruch 3, in welcher die Kapillarröhrchen freigabefähig durch Reibungsbefestigung an einem nach aussen gerichteten Schlitz oder einer Arretierung an der Peripherie der Spule oder des Karussels festgehalten werden.

5. Vorrichtung gemäss Anspruch 4, umfassend wieterhin Einrichtungen (61) zum Beladen von einzelnen Kapillarröhrchen in die einzelnen Schlitze oder Arretierungen der Spule oder des Karussels, bevor die Röhrchen zu den Betriebsstationen geleitet werden, und Einrichtungen zum Ausladen der Kapillarröhrchen von der Spule oder dem Karussel nach Beendigung des Immunoassays.

6. Vorrichtung gemäss einem der Ansprüche 1 bis 5, umfassend weiterhin Einrichtungen, um die Vielzahl von Proben zuzuführen, wobei die Einrichtungen eine Vielzahl von Probeverteilungsbechern umfassern, die in Becherfördereinrichtungen (62) befestigt sind und Antriebseinrichtungen, um jeden der Probeausgabebecher in Reihenfolge zu der ersten Betriebsstation (I) wieterzuführen, wobei jeder der Probeabgabebecher eine kleine Öffnung an der Basis hat und die Probe darin durch Kapillarwirkung festgehalten wird, wobei die Vorrichtung weiterhin Beladeeinrichtungen (63) bei der ersten Betriebsstation umfasst, um jedes Kapillarröhrchen in Reihenfolge mit der Basis eines jeweiligen der Abgabebecher zu kontaktieren, so dass die Probe in dem Abgabebecher in das Kapillarrohr eingebracht wird.

7. Vorrichtung gemäss einem der Ansprüche 1 bis 6, weiterhin umfassend Wasch- (100) und/oder Vakuumtrocknungs- (120) Einrichtungen, die an ausgewählten Stellen (VI) längs des Pfades der Kapillarröhrchen zwischen den Betriebsstationen angeordnet sind.

8. Vorrichtung gemäss Anspruch 7, worin jeder der Wasch- und/oder Vakuumtrockeneinlagen eine Kolbeneinrichtung (101), die innerhalb eines Gehäuses (102) beweglich ist, umfasst, um jedes der Kapillarröhrchen in Reihenfolgen, wenn es die Wasch- und/oder Vakuumtrockeneinrichtung passiert, mit einer Waschflüssigkeit zu dem Röhrchen zu versehen und/oder die Wasch- und Testflüssigkeiten aus dem Röhrchen zu entfernen.

9. Vorrichtung gemäss einem der Ansprüche 1 bis 8, in welcher die Kapillarröhrchen eine Kapazität von 1 bis 30 µl, vorzugsweise 10 bis 15 µl, haben.

10. Vorrichtung zur Durchführung von automatisierten, heterogenen Enzym-Immunoassays für den Nachweis oder die Bestimmung von antigenischen oder haptenischen Substanzen oder Antikörpern in einer Mehrzahl von Proben, umfassend:

(a) eine Mehrzahl von Kapillarröhrchen (12), wobei jedes der Kapillarörchen Antikörper oder antigenische oder haptenische Substanzen an seiner inneren Oberfläche befestigt enthält,

(b) Einrichtungen (13), um jedes der Kapillarrörchen nacheinander zu einer Mehrzahl von Betriebsstationen zu führen,

(c) Einrichtungen (63) bei einer ersten Betriebsstation (I), um die einzelnen Proben der Reihe nach zu jedem der Kapillarröhrchen zuzuführen, wenn diese die erste Betriebsstation passieren,

(d) Einrichtungen (65), um ein Enzymkonjugat zu jeder der Proben zuzuführen, wobei das Enzymkonjugat entweder zu jedem der einzelnen Proben zugegeben wird bevor die Probe in eines der Kapillarröhrchen eingebracht wird, oder zu jeder der einzelnen Proben in einem der Kapillarröhrchen in einer zweiten Betriebsstation (II) zugegeben wird,

(e) Einrichtungen (66) bei einer dritten Betriebsstation (III) zum Einbringen eines Enzymsubstrats in jedes der Kapillarröhrchen, wenn diese die dritte Betriebsstation passieren, und

(f) Einrichtungen bei einer vierten Betriebsstation (IV) zum Nachweis oder zur Bestimmung einer Enzymwirkung, falls eine solche vorhanden ist, bei dem Enzymsubstrat in jedem der Kapillarröhrchen.

11. Verfahren zur Durchführung von automatisierten heterogenen Immunoassays für den Nachweis oder die Bestimmung von antigenischen oder haptenischen Substanzen oder von Antikörpern in einer Mehrzahl von Proben, umfassend:

(a) das Zurverfügungstellen einer Mehrzahl von Kapillarröhrchen, wobei jedes der Kapillarröhrchen Antikörper oder antigenisch oder haptenische Substanzen an seiner inneren Oberflächen befestigt enthält,

(b) Passieren eines jeden der Kapillarröhrchen der Reihe nach zu einer Mehrzahl von Betriebsstationen,

(c) Zugabe von einzelnen Proben, der Reihe nach, zu jedem der Kapillarröhrchen bei einer ersten Betriebsstation,

(d) Zugabe eines Immunoassay-Reagens zu jedem der Kapillarröhrchen an einer oder mehreren nachfolgenden Betriebsstationen, und

(e) Nachweis oder Bestimmung des Ergebnisses des Immunoassays bei jedem der Kapillarröhrchen in einer letzten Betriebsstation.

12. Verfahren zur Durchführung von automatisierten heterogenen Enzym-Immunoassays für den Nachweis oder zur Bestimmung von antigenischen oder haptenischen Substanzen oder von Antikörpern in einer Mehrzahl von Proben, umfassend:

(a) Zurverfügungstellen einer Mehrzahl von Kapillarröhrchen, wobei jedes der Kapillarröhrchen Antikörper oder antigenische oder haptenische Substanzen an seiner inneren Oberfläche befestigt enthält,

(b) Passieren der Kapillarröhrchen der Reihe nach zu einer Mehrzahl von Betriebsstationen,

(c) Zugabe von einzelnen Proben der Reihe nach zu jedem der Kapillarröhrchen bei einer ersten Betriebsstation,

(d) Zugabe von Enzymkonjugat zu jeder der Proben und zwar entweder bevor die Probe zu dem Kapillarröhrchen gegeben wird oder bei einer zweiten Betriebsstation,

(e) Zugabe von Enzymsubstrat zu jedem der Kapillarröhrchen bei einer dritten Betriebsstation, und

(f) Nachweis oder Bestimmung der Enzymwirkung, falls vorhanden, bei dem Enzymsubstrat in jedem der Kapillarröhrchen bei einer vierten Betriebsstation.

**Revendications**

1. Appareil pour effectuer des analyses immunologiques hétérogènes automatisées pour la détection ou la détermination de substances antigéniques ou hapténiques ou d'anticorps dans plusieurs échantillons, qui comprend:

(a) plusieurs tubes capillaires (12), chacun desdits tubes capillaires ayant des anticorps ou des substances antigéniques ou hapténiques fixés à sa surface interne;

(b) un dispositif (10, 13) pour amener successivement chacun desdits tubes capillaires à plusieurs postes opératoires;

(c) un dispositif à un premier poste opératoire (I) pour introduire successivement des échantillons individuels dans chacun desdits tubes capillaires lorsqu'il est amené audit premier poste opératoire;

(d) un dispositif (65, 66) en un ou plusieurs postes opératoires ultérieurs (II, III) pour introduire un réactif d'analyse immunologique dans chacun desdits tubes capillaires lorsqu'il est amené audit ou auxdits poste(s) opératoire(s) ultérieur(s); et

(e) un dispositif en une poste opératoire final (IV, V) pour détecter ou déterminer le résultat de l'analyse immunologique dans chacun desdits tubes capillaires lorsqu'il est amené audit poste opératoire final.

2. Appareil comme revendiqué dans la revendication 1 dans lequel lesdits tubes capillaires (12) sont montés sur un support (11), ledit support comprenant une bobine ou plateau tournant ayant lesdits tubes capillaires montés verticalement sur son pourtour.

3. Appareil comme revendiqué dans la revendication 2 dans lequel ledit dispositif pour conduire (10, 13) chacun desdits tubes capillaires à plusieurs opérations comprend un dispositif (13) pour faire tourner ladite bobine ou ledit plateau tournant par étapes.

4. Appareil comme revendiqué dans la revendication 2 ou la revendication 3 dans lequel chacun desdits tubes capillaires est maintenu de façon amovible par engagement par friction dans une encoche ou cliquet dirigé vers l'extérieur au pourtour de ladite bobine ou dudit plateau tournant.

5. Appareil comme revendiqué dans la revendication 4 comprenant de plus un dispositif (61) pour charger les tubes capillaires individuels dans les encoches ou cliquets individuels de ladite bobine ou dudit plateau tournant avant le passage desdits tubes auxdits postes opératoires et un dispositif pour décharger lesdits tubes capillaires de ladite bobine ou dudit plateau tournant lors de l'achèvement de l'analyse immunologique.

6. Appareil comme revendiqué dans l'une quelconque des revendications 1 à 5 comprenant de plus un dispositif pour fournir lesdits plusieurs échantillons, ledit dispositif comprenant plusieurs cupules de distribution d'échantillons montées dans un dispositif de transport de cupules (62) et un dispositif d'entraînement pour faire avancer successivement chacune desdites cupules de distribution d'échantillons audit premier poste opératoire (I) chacune desdites cupules de distribution d'échantillons ayant une petite ouverture dans sa base et l'échantillon y étant retenu par capillarité, l'appareil comprenant de plus un dispositif de chargement (63) audit premier poste opératoire pour mettre successivement chaque tube capillaire en contact avec la base d'une desdites cupules de distribution correspondantes pour que l'échantillon dans ladite cupule de distribution soit chargé dans ledit tube capillaire.

7. Appareil comme revendiqué dans l'une quelconque des revendications 1 à 6 comprenant de plus des dispositifs de lavage (100) et/ou de séchage sous vide (120) placés en des points choisis (VI) le long du trajet des tubes capillaires entre lesdits postes opératoires.

8. Appareil comme revendiqué dans la revendication 7 dans lequel chacun desdits dispositifs de lavage et/ou se séchage sous vide comprend un dispositif à piston (101) mobile dans un logement (102) pour venir successivement en contact avec chacun desdits tubes capillaires lorsqu'il est amené au dit dispositif de lavage et/ou de séchage sous vide pour introduire un fluide de lavage dans ledit tube et/ou pour éliminer les fluides de lavage et d'analyse dudit tube.

9. Appareil comme revendiqué dans l'une quelconque des revendications 1 à 8 dans lequel chacun desdits tubes capillaires a une capacité de 1 à 30 µl, de préférence de 10 à 15 µl.

10. Appareil pour effectuer des analyses immuno-enzymatiques hétérogènes automatisées pour la détection ou la détermination de substances antigéniques ou hapténiques ou d'anticorps dans plusieurs échantillons qui comprend:

(a) plusieurs tubes capillaires (12) chacun desdits tubes capillaires ayant des anticorps ou des substances antigéniques ou hapténiques fixés à sa surface interne;

(b) un dispositif (13) pour amener successivement chacun desdits tubes capillaires à plusieurs postes opératoires;

(c) un dispositif (63) en un premier poste opératoire (I) pour introduire successivement les échantillons individuels dans chacun desdits tubes capillaires lorsqu'il est amené audit premier poste opératoire;

(d) un dispositif (65) pour introduire un conjugué enzymatique dans chacun desdits échantillons, ledit conjugué enzymatique étant ajouté soit à chacun desdits échantillons individuels avant que ledit échantillon soit introduit dans un desdits tubes capillaires, soit à chacun desdits échantillons individuels dans un desdits tubes capillaires en un second poste opératoire (II);

(e) un dispositif (66) en un troisième poste opératoire (III) pour introduire un substrat enzymatique dans chacun des dits tubes capillaires lorsqu'il est amené audit troisième poste opératoire; et

(f) un dispositif en un quatrième poste opératoire (IV) pour détecter ou déterminer une action enzymatique éventuelle sur ledit substrat enzymatique dans chacun desdits tubes capillaires.

11. Un procédé pour effectuer des analyses immunologiques hétérogènes automatisées pour la détection ou la détermination de substances antigénique ou hapténiques ou d'anticorps dans plusieurs échantillons qui consiste à:

(a) se pourvoir de plusieurs tubes capillaires, chacun des dits tubes capillaires ayant des anticorps ou des substances antigéniques ou hapténiques fixés à sa surface interne;

(b) amener successivement chacun desdits tubes capillaires à plusieurs postes opératoires;

(c) introduire successivement des échantillons individuels dans chacun desdits tubes capillaires en un premier poste opératoire;

(d) introduire un réactif d'analyse immunologique dans chacun desdits tubes capillaires en un ou plusieurs postes opératoires ultérieurs; et

(e) détecter ou déterminer le résultat de l'analyse immunologique dans chacun desdits tubes capillaires en un poste opératoire final.

12. Un procédé pour effectuer des analyses immuno-enzymatiques hétérogènes automatisées pour la détection ou la détermination de substances .antigéniques ou hapténiques ou d'anticorps dans plusieurs échantillons qui consiste à:

(a) se pourvoir de plusieurs tubes capillaires, chacun des dits tubes capillaires ayant des anticorps ou des substances antigéniques ou hapténiques fixés à sa surface interne;

(b) amener successivement chacun desdits tubes capillaires à plusieurs postes opératoires;

(c) introduire successivement les échantillons individuels dans chacun desdits tubes capillaires à un premier poste opératoire;

(d) ajouter un conjugué enzymatique à chacun desdits échantillons, soit avant qui ledit échantillon soit introduit dans un tube capillaire, soit à un second poste opératoire;

(e) introduire un substrat enzymatique dans chacun desdits tubes capillaires en un troisième poste opératoire; et

(f) détecter ou déterminer une action enzymatique éventuelle sur ledit substrat enzymatique dans chacun desdits tubes capillaires à un quatrième poste opératoire.

Fig. I.

Fig. Ia.

Fig. Ib.

FIG. 2.

0 089 346

FIG. 3.